# EUROPÄISCHE PATENTSCHRIFT

(11) **EP 2 041 172 B1**
(45) Veröffentlichungstag und Bekanntmachung des Hinweises auf die Patenterteilung: **27.11.2013**
(21) Anmeldenummer: 07765068.7
(22) Anmeldetag: 05.07.2007
(51) Int. Cl.: C07K 14/705, C12N 5/10, G01N 33/50

(54) **ZELLULÄRER PYROGENTEST MITTELS TOLL-LIKE REZEPTOR**
CELLULAR PYROGENIC TEST USING TOLL-LIKE RECEPTOR
TEST CELLULAIRE DE RECHERCHE DES PYROGÈNES AU MOYEN D'UN RÉCEPTEUR DE TYPE TOLL

(30) Priorität: 07.07.2006 DE 102006031483
(43) Veröffentlichungstag der Anmeldung: 01.04.2009
(73) Patentinhaber: Fraunhofer-Gesellschaft zur Förderung der angewandten Forschung e.V., 80686 München (DE)
(72) Erfinder: BRUNNER, Herwig, 82362 Weilheim (DE); FINKELMEIER, Doris, 70374 Stuttgart (DE); GEIGER, Georg, 71032 Böblingen (DE); BURGER-KENTISCHER, Anke, 70569 Stuttgart (DE)
(74) Vertreter: Schwahn, Hartmut
(86) Internationale Anmeldenummer: PCT/EP2007/005946
(87) Internationale Veröffentlichungsnummer: WO 2008/003489

(56) Entgegenhaltungen:
- WO-A-2007/006095
- WO-A-2007/076411
- JP-A- 2004 357 607
- US-A1- 2003 232 055
- US-A1- 2004 197 865
- TOSI ET AL: "Innate immune responses to infection" JOURNAL OF ALLERGY AND CLINICAL IMMUNOLOGY, MOSBY - YEARLY BOOK, INC, US, Bd. 116, Nr. 2, August 2005 (2005-08), Seiten 241-249, XP005002803 ISSN: 0091-6749

## Beschreibung

Die Erfindung betrifft Verfahren, mittel und Kits zum qualitativen und quantitativen Nachweis und Identifikation von Erregern und Erregerspektren auf Basis von Endotoxinen und anderen Pyrogenen.

### Stand der Technik

Von großer Bedeutung für die Einleitung einer zielgerichteten Infektionstherapie, z. B. bei Sepsispatienten ist eine schnelle und sichere Identifikation der Erregerspektren in der klinischen Diagnostik in Krankenhäusern.

Sepsis und Sepsis assoziierte Multiorganversagen sind weltweit die wichtigsten Letalitätsfaktoren und gehören zu den ungelösten Problemen der Medizin. Nach vorsichtigen Schätzungen sterben derzeit jährlich immer noch weltweit rund 500.000 Patienten an den Folgen einer "Blutvergiftung", das sind 1.400 pro Tag. Die jährliche Belastung des Gesundheitsbudgets durch die Behandlung von Patienten mit schwerer Sepsis wurde in den USA auf 17 Millionen Dollar geschätzt. Als Sepsis (Septikämie, Blutvergiftung) wird die Summe lebensbedrohlicher Krankheitssymptome und pathophysiologischer Veränderungen bezeichnet. Sie wird durch pathogene Keime und ihre Produkte verursacht, die aus einem Infektionsherd in die Blutbahn eindringen. Die darauf einsetzende Immunreaktion führt zur Bildung von endogenen Mediatoren (Zytokinen). Dies aktiviert die Entzündungskaskade und es kommt in Folge zu einer nicht mehr kontrolliert ablaufenden systemischen Entzündungsreaktion. Trotz intensivmedizinischer Maßnahmen ist die Prognose ernst; die Letalität beträgt um 50 %. Besonders ungünstig ist die Prognose bei spätem Therapiebeginn, nicht lokalisierbarem Infektionsherd oder nicht identifizierbarem Erreger.

Sepsis auslösende Erreger sind meist Bakterien, vorwiegend gramnegative Bakterien wie E. coli, andere Enterobacterien, Klebsiella-, Proteus-, Enterobacter-Arten, Pseudomonas aeruginosa, Neisseria meningitidis und Bacteroides aber auch häufiger grampositive Bakterien wie Staphylococcus aureus, Streptococcus pneumoniae und andere Streptokokken; seltener Pilze, Viren oder Parasiten (Bakteriämie, Fungämie, Virämie, Parasitämie). Durch Freisetzung von so genannten mikrobiellen Strukturen (z.B. Endotoxine, Exotoxine, Superantigene) wird die Ausschüttung von endogenen Mediatoren, wie Interleukinen, Tumornekrosefaktoren, Histamin, Serotonin, Sauerstoffradikalen und Proteasen, stimuliert. Diese führen durch Aktivierung von Leukozyten und humoralen Abwehrsystemen zu den für den septischen Schock typischen Veränderungen. Die systemische Entzündung als Reaktion auf zirkulierende mikrobielle Antigene ist ein wesentliches Charakteristikum der Pathophysiologie von Sepsis und septischem Schock. Bei Kontakt der Zellen der unspezifischen Immunabwehr mit Lipopolysaccharid (LPS), Bestandteilen der bakteriellen Zellwand, Peptidoglykanen oder Lipoteichonsäure (LTA) wird die angeborene Immunität aktiviert, und es werden in der frühen Phase der Infektion von verschiedenen Immunzellen Zytokine sezerniert. Obwohl diese Zytokine eine wichtige Rolle in der Abwehrreaktion spielen, es werden beispielsweise aktivierte Neutrophile zum Entzündungsort gelockt, führt das Eintreten dieser Zytokine und bakterieller Substanzen in den Blutkreislauf zu einer Kette von ungünstigen pathophysiologischen Ereignissen. Der mit dieser Entzündungsreaktion einhergehende klinische Symptomkomplex wird als Systemic Inflammatory Response Syndrome (SIRS) bezeichnet.

Bei Vorliegen einer septischen Erkrankung oder auch schon bei Verdacht auf eine solche Erkrankung muss eine Therapie frühzeitig erfolgen. Bislang gibt es keine Möglichkeit, das Erregerspektrum eines Sepsispatienten schnell und zuverlässig zu identifizieren. Die herkömmliche Diagnose der Sepsis besteht in einer meist mehrfachen Abnahme einer klinischen Probe: Blut- und Urinkultur, Sputum, Stuhl, Wundsekreten zur Erregeridentifizierung mit Resistenzbestimmung vor Beginn der Antibiotikatherapie. Die Wahrscheinlichkeit, mit den bisherigen Methoden einen Erregernachweis bei einer "Septikämie" zu führen liegt bei 30 bis 50%. Dies kann nur durch das Anlegen von mehreren Kulturen für mikrobiologische Untersuchungen, Keimanzüchtungsversuche aus einer venösen Blutprobe oder aus Urin, erreicht werden Die Probe wird in flüssiges Nährmedium verimpft und bebrütet. Dieses Verfahren kostet wertvolle Zeit, führt oft nicht zur Identifikation der Erreger, da es nur bei vitalen Erregern möglich ist: Wurde die Probe während einer Antibiotikatherapie entnommen, sind die Züchtungsversuche meist erfolglos. Es wird daher immer noch eine breit angelegte antibiotische, antimykotische, antivirale und/oder antiparasitäre Therapie angewandt. Pyrogene Stoffe der Erreger wie Zellwandbestandteile können mit dieser Methode nicht erfasst werden.

Es besteht deshalb der Bedarf an einem schnellen und einfachen Testsystem, das den Nachweis und die Differenzierung des Sepsiserregers oder -erregerspektrums ermöglicht.

Pyrogene sind fiebererzeugende Stoffe, so genannte pyrogene Substanzen, die zur Phagozytose befähigte Körperzellen (Immunzellen) zur Synthese von proinflammatorischen Interleukinen (vor allem IL-1 und IL-6) und Tumornekrosefaktor-a (TNF-a) anregen, die dann als "eigentliche Pyrogene" das Temperaturzentrum des Körpers so beeinflussen, dass es zu erhöhter Wärmeproduktion und verminderter Wärmeabgabe kommt. Die am stärksten wirksamen Pyrogene stammen von gramnegativen Bakterien. Bei den Pyrogenen handelt es sich nicht um eine einheitliche Substanzgruppe. Dazu zählen Zellwandbestandteile und Stoffwechselprodukte von Mikroorganismen (apathogene und pathogene Bakterien, Pilze und Viren) sowie Parasiten, z. B. Endotoxine, Exotoxine oder Supreantigene.

Klinische Bedeutung haben Pyrogene vor allem bei Injektion bzw. Infusion pyrogenhaltiger Flüssigkeiten, wie Stabilisatorlösungen, bei Verwendung bakteriell verunreinigter Blutkonserven, nicht pyrogenfreier Injektionsspritzen, Infusionsgeräte usw. Fehlende Pyrogenfreiheit ist die Hauptursache für so genannte "Transfusionszwischenfälle", die mit hohem Fieber, Schock, Verbrauchskoagulopathie und akutem Nierenversagen einhergehen. Besonders sind heute zusätzliche Risikofaktoren, über die Pyrogene in den Körper gelangen können, wie zentralvenöse Katheter, langfristige Sondenernährung und Langzeitbeatmung hinzugekommen.

Pyrogene sind meist hitzebeständige und dialysierbare Stoffe, z.B. Lipopolysaccharid-Protein-Lipid-Komplexe, LPS. Deshalb reichen übliche Verfahren zur Sterilisierung von Infusionslösungen, Instrumenten und Geräten, die für den Einsatz am menschlichen oder tierischen Körper gedacht sind, nicht aus, um diese von pyrogenen Substanzen zu befreien. Zusätzliche Reinigungsschritte sind erforderlich. Die Pyrogenfreiheit ist eine zwingende Voraussetzung für den Einsatz solcher Produkte am Körper. Weiter sollten alle Erzeugnisse, die in intensivem Kontakt mit dem menschlichen oder tierischen Organismus kommen, sei es weil sie in die Blutbahn appliziert werden, sei es weil sie lange im Körper verweilen, ausreichend pyrogenfrei sein.

Das Spektrum der auftretenden pyrogenen Substanzen hängt vom Erreger oder Erregerspektrum ab. Bestimmte Erreger bilden erregertypische bzw. erregerspezifische Pyrogenmuster, so genannte "pathogen-associated microbial patterns" oder PAMPs. Anhand der Identifikation und Differenzierung der PAMPs könnte eine Zuordnung zu einem bestimmen Erreger oder Erregerspektrum erfolgen.

Um pyrogene bzw. PAMPs in einer Probe nachzuweisen (Pyrogen-Test), stehen heute vor allem drei kommerziell verwertete Nachweismethoden oder Tests zur Verfügung. Ein bekannter Test ist der Kaninchen-Pyrogen-Test. Er beruht auf der "Fieberreaktion" der Tiere auf Pyrogene. Es handelt sich um einen Tierversuch, bei welchem den Kaninchen die Prüfsubstanz in die Ohrvene appliziert wird. Um eine Abwehrreaktion des tierischen Körpers gegen die Substanz nachzuweisen wird nach mehreren Stunden rektal Fieber gemessen. Diese Test ist zeit- und kostenintensiv und mit dem kalkulierten Leiden von Tieren verbunden. Endotoxine und Nicht-Endotoxin-Pyrogene können damit detektiert aber nicht identifiziert werden. Ein Test auf Viren ist nicht möglich. Eine Spezifizierung der PAMPs ist nicht möglich. Außerdem ist seine Übertragbarkeit auf den Menschen umstritten.

Ein weiterer bekannter Test ist der "Limulus Amöbocyten Lysat Test" (LAL) (z.B. Fa. Cambrex Bioscience oder Fa. Charles River). Er beruht auf der Abwehrreaktion von Arthropoden gegen bestimmte als Pyrogene bekannte Substanzen. Beim LAL wird aus Blutzellen des Limulus polyphemus ein Proenzym gewonnen, welches über gramnegatives bakterielles Endotoxin in ein aktives Enzym umgesetzt wird. Durch eine Enzym-Substrat Umsetzung kann die Endotoxinmenge quantitativ, z.B. über Photometer, bestimmt werden. Dieses Verfahren ist sensitiver und besser standardisierbar als der bekannte Kaninchentest, aber es erfasst nur Endotoxine gramnegativer Organismen (z.B. Lipopolysaccharid, LPS; Nachweisgrenze: 3 pg/ml). Solche Endotoxine stellen nur einen geringen Teil der bekannten pyrogenen Stoffe dar. Andere Pyrogene bleiben unerkannt. In den letzten Jahren haben aber gerade gram-positive Erreger gegenüber den gram-negativen Bakterien zunehmend an Bedeutung gewonnen.

Ein weiterer bekannter Test ist schließlich der Immun-Pyrogen-Test, z.B. "Endosafe - IPT" (Fa. Charles River). Er beruht auf der "Fieber-reaktion" humaner Zellen auf vorhandene Pyrogene. Es handelt sich um einen humanen Vollbluttest, bei welchem als Antwort auf einen pyrogenen Stoff aus vitalen Blutzellen das Zytokin IL-1 ausgeschüttet wird, das mittels ELISA quantitativ bestimmt werden kann (Nachweisgrenze: 20-50 pg/ml). Dieses System erfasst auch Pyrogene gram-positiver Erreger. Der Test ist jedoch mit noch größerem Zeit- und Arbeitsaufwand verbunden. Menschliches Vollblut muss bereitgestellt werden, welches potentiell humanpathogen ist. Eine Spezifikation der PAMPs ist nicht möglich.

Die bekannten Tests sind zeitaufwändig und setzen ein apparativ gut ausgerüstetes Labor (ELISA-Test, Humanblutverarbeitung, Tierexperiment) voraus. Es besteht deshalb der Bedarf an einem schnell und einfach durchzuführenden Testsystem zum Nachweis von Pyrogenen. Es besteht außerdem der Bedarf an einem Testsystem zur Spezifizierung des Pyrogenmusters PAMP, um daraus Rückschlüsse auf den Erreger oder das Erregerspektrum ziehen zu können. Dies ist vorteilhaft für die Diagnose und Therapie von Infektionskrankheiten, bei denen das Auftreten von Pyrogenen eine Rolle spielt, allen voran die Sepsis.

Weiter besteht Bedarf an verbesserten Tests auf pyrogene Rückstände an medizinischen Geräten, Spendergewebe, injizierbaren Arzneimitteln und Medizinprodukten wie Implantate oder Instrumente (Katheter, etc.). Auch in der Lebensmittelindustrie und Pharmaindustrie besteht der Bedarf an verbesserten Nachweisen von pyrogenen Substanzen und Keimen und deren Identifikation in Nahrungsmitteln, Nahrungsmittelbestandteilen, Roh- und Ausgangsstoffen für Nahrungs- oder Arzneimittel.

Es ist bekannt, dass so genannte "Toll-like Rezeptoren" (TLR, TLRs) im Zusammenhang mit pathophysiologischen Vorgängen bei der Sepsis und ähnlichen Infektionskrankheiten, die mit dem Auftreten von Pyrogenen im Organismus einhergehenden, in Verbindung stehen. TLRs vermitteln die körperlichen Reaktionen auf Pyrogene. Bei einer mikrobiell ausgelösten Sepsis stimulieren bakterielle Bestandteile die Immunzellen des Wirtes über die TLRs.

TLRs sind hoch konservierte Transmembranproteine mit leucinreichen extrazellulären Domänen und einer zytoplasmatischen Domäne aus ungefähr 200 Aminosäuren. Sie gehören aufgrund ihrer Homologie in der Zytoplasmadomäne zur Interleukin-1-Rezeptor/Toll-like-Rezeptor-Superfamilie. Die charakteristische zytoplasmatische TIR-Domäne ist für die Signalübertragung essentiell. Die Extrazellulärdomäne ist direkt an der Erkennung der unterschiedlichen pathogenen Molekülstrukturen beteiligt und unterscheidet sich stark von der des IL-1 Rezeptors. Während der Extrazellulärteil des IL-1 Rezeptors aus drei Immunglobulindomänen besteht, besitzen TLRs 18-26 LRR von je 24-29 Aminosäuren Länge. TLRs werden im Gegensatz zu dem aus Drosophila bekannten Protein "Toll" direkt von Fremdstrukturen aktiviert. Bis heute sind zehn verschiedene humane TLRs und 13 TLRs der Maus identifiziert worden. Sie werden auf verschiedenen Zelltypen des Immunsystems exprimiert, überwiegend auf Monozyten, Makrophagen, dendritischen Zellen sowie B- und T-Zellen. TLRs sind auf der Plasmamembran lokalisiert; TLR-3, TLR-7 und TLR-9 werden von Nukleinsäure-Motiven aktiviert und sind in intrazellulären Kompartimenten zu finden.

TLR-2 ist essentiell für die Erkennung einer Vielzahl an PAMPs aus grampositiven Bakterien, einschließlich bakterieller Lipoproteine und Lipoteichonsäuren. TLR-3 ist in die Erkennung doppelsträngiger Virus-RNA involviert. TLR-4 wird überwiegend durch LPS aktiviert. TLR-5 detektiert bakterielles Flagellin. TLR-7 und TLR-8 erkennen synthetische kleine antivirale Moleküle und einzelsträngige RNA. TLR-9 wurde im Endoplasmatischen Retikulum (ER) nachgewiesen und wird nach Stimulation mit CpG-Motive enthaltener DNA, zum Beispiel CpG-Oligodeoxynucleotide, in die endosomalen/lysosomalen Kompartimente rekrutiert. CpG-Motive sind Bereiche innerhalb eines Nukleinsäure-Stranges, in denen die Bausteine Cytosin (C) und Guanin (G) unerwartet häufig vorkommen ('p' steht für eine Phosphatgruppe, die beide Bausteine ,C' und ,G' verbindet); solche CpG-Motive finden sich besonders häufig im Genom von Bakterien und Viren, nicht aber von Wirbeltieren.

Antagonisten der Toll-like Rezeptoren finden zunehmend Einsatz in der Dermatologie z.B. zur Behandlung von virusinduzierten Papillomen. Es besteht der Bedarf an einem Testsystem zum Screenen neuer TLR Antagonisten.

Das Konzept der Aktivierung des humanen Immunsystems steht im Interesse bei der Krebstherapie. Substanzen wie CPG 7909 (Fa. Coley Pharmaceutical Group) führen auf diese Weise immunmodulatorische Effekte herbei und können damit die Wirksamkeit von Chemotherapien verbessern. Es besteht der Bedarf an einem Testsystem zum Screenen neuer CpG-Motive (Oligodeoxynucleotide).

Aus der WO 2005/077408 sind NIH 3T3-Zellen bekannt, welche Toll-like Rezeptoren exprimieren und ein NF-κB-Reportersystem enthalten. Die WO 01/032877 beschreibt die Transfektion von murinen Toll-like Rezeptoren in die Zelllinie NIH 3T3 zusammen mit dem NF-κB-Luciferase-Reportergenassay um CpG-Oligonukleotide zu erkennen. Aus der JP 2004-357607 A ist ein Pyrogentestsystem mit HEK293-Zellen, in die Toll-like Rezeptoren vom Typ 4 transformiert wurden, bekannt.

Der vorliegenden Erfindung liegt vor allem das technische Problem zugrunde, Verfahren und Mittel zum spezifischen Nachweis von Pyrogenen (spezifischer Pyrogentest) bereitzustellen. Damit verbunden besteht ein weiteres technisches Problem in der Bereitstellung von Verfahren und Mitteln zum spezifischen Nachweis von Erregern oder Erregerspektren bei Infektionen des menschlichen oder tierischen Körpers. Damit verbunden besteht ein weiteres technisches Problem in der Bereitstellung von Verfahren und Mitteln zum Screenen neuer TLR Antagonisten und/oder neuer CpG-Motive.

Das technische Problem wird gelöst durch die Bereitstellung einer transgenen Zelle oder Zelllinie zum spezifischen Nachweis eines Pyrogens in einer Probe mit den kennzeichnenden Merkmalen nach Anspruch 1. Die Zelle liegt vorzugsweise adhärent vor. In einer Variante liegt die Zelle vorzugsweise in Suspension vor.

Erfindungsgemäß weist die transgene Zelle oder Zelllinie im Genom (a) mindestens ein Gen oder Gene, die mindestens für einen humanen Toll-like Rezeptor (TLR) Typ 2 (TLR-2) und/oder den humanen TLR Typ 4 (TLR-4) kodieren, die die Zelle exprimiert, und (b) mindestens ein Reportergen, das unter der Expressionskontrolle eines durch NF-κB induzierbaren Promotors steht, auf. Bevorzugt weist die Zelle oder Zelllinie im Genom zusätzlich ein Gen auf, das für den CD14-Rezeptor kodiert. Die erfindungsgemäße Zelle oder Zellline ist die murinen Fibroblastenzelle vom Typ NIH-3T3. Bevorzugt werden die TLR, besonders zusammen mit dem Corezeptor CD14 (MD2), über Plasmide transfiziert.

Die Erfindung sieht also vor, eine transgene Fibroblastenzelle NIH-3T3, bereitzustellen, die zumindest humanen TLR-2 oder humanen TLR-4 exprimiert und bevor zugt den CD14-Corezeptor co-exprimiert. Die Erfinder fanden überraschend, dass diese transgene Zelle in Kontakt mit Pyrogenen, die spezifisch den exprimierten TLR aktivieren, die vom Reportergen kodierte Enzymaktivität exprimiert, die beispielsweise durch Farbreaktion nachgewiesen und unter bestimmten Voraussetzungen quantifiziert werden kann. Es wird also eine zelluläres Testsystem zum Nachweis von Pyrogenen, PAMPs und anderen TLR aktivierenden Wirkstoffen bereitgestellt.

Die Selektivität und die Sensitivität dieses Testsystems ist hoch. Die Sensitivität liegt bei etwa 1 bis 10 pg/ml LPS. Im Vergleich dazu beträgt die Sensitivität der herkömmlichen Pyrogentestsysteme etwa 3 bis 10 pg/ml (LAL) oder 20-50 pg/ml (IPT).

Das erfindungsgemäße Testsystem kann ohne die apparative Ausstattung eines Zellkulturlabors wie CO₂-Begasung etc. auskommen und ist damit für jeden Anwender auch ohne spezielle Laborausrüstung einfach anzuwenden.

Das prinzipielle durch die erfindungsgemäße Lehre charakterisierte Testverfahren kann auf Zelllinien für alle TLRs (z. B. humane TLRs 1-10) erweitert werden, so das damit alle PAMPs selektiv erkannt und identifiziert werden können. So kann vorteilhafterweise ein einfaches und schnelles zelluläres Testsystem bereitgestellt werden, das einen spezifischen Nachweis eines oder mehrerer Pyrogene oder (pathogen-associated microbial patterns) PAMPs sowie deren Quantifizierung erlaubt. Ohne an die Theorie gebunden zu sein, induziert Aktivierung von TLRs Signaltransduktionswege, die mit Hilfe des Transkriptionsfaktors NF-κB zur Produktion verschiedener Zytokine führt. An der Signalkaskade sind eine Vielzahl an Proteinen involviert wie das MyD88 und IRAK1. Diese Signalkaskade führt über die Aktivierung des Transkriptionsfaktors NF-κB zur Induktion und Produktion proinflammatorischer Zytokine. Die Zytokine Tumornekrosefaktor (TNF), Interleukin-1 (IL-1) und Interleukin-6 (IL-6) gelten als die wichtigsten zentral und initial involvierten Mediatoren bei diesem Prozess. Nach der Aktivierung der TLRs kommt es zur Rekrutierung von Adaptormolekülen und zur Produktion proinflammatorischer Zytokine. Die Sekretion dieser Zytokine führt zu einer Stimulierung des Immunsystems und verteidigt gegen den eindringenden Mikroorganismus. Jedoch kann eine stark überschießende Produktion zur Sepsis bzw. zum septischen Schock führen. Im Zusammenhang mit der Diagnose der Sepsis sind vor allem die TLR-2 und TLR-4 bevorzugt. Während TLR-2 Bestandteile grampositiver Bakterien wie Peptidoglykane, Lipopeptide und LTA erkennt, ist TLR-4 der Rezeptor für LPS, dem Hauptbestandteil der Zellwand gramnegativer Bakterien. TLR-2 und TLR-4 stehen also bei grampositiver und gramnegativer Sepsis als Signal übertragende Rezeptoren im Mittelpunkt und sollen deshalb für die Differenzierung des Erregerspektrums bevorzugt herangezogen werden. Tabelle 1 zeigt die Spezifitäten der einzelnen humanen TLRs.

**Tabelle 1:**

| **Ligand/Pyrogen** | **Herkunft** | **TLR-Typ** |
|---|---|---|
| Zellwandbestandteile (Peptidoglykan, Lipopeptide, Lipoteichonsäuren) | Bakterien | TLR 1 /TLR 2 (Heterodimer) |
| Lipoproteine | Bakterien | TLR 2 |
| Lipopeptide | Mycoplasmen | TLR 2 /TLR 6 (Heterodimer) |
| Zymosan | Hefen, Pilze | TLR 2 /TLR 6 (Heterodimer) |
| doppelsträngige RNA | Viren | TLR 3 |
| Lipopolysaccharide (LPS) | gramnegative Bakterien | TLR 4, CD14 |
| Heatshockprotein 60 (Hsp 60) | Mensch/Pilze | TLR 4 |
| Flagellin | Bakterien | TLR 5 |
| einzelsträngige RNA | Viren | TLR 7/TLR 8 (Heterodimer) |
| unmethylierte CpG-Motive | Bakterien, Viren | TLR 9 |

Durch die Identifikation der erregerspezifischen PAMPs ergibt sich die Möglichkeit einer schnellen Identifikation Sepsis auslösender Keime. Mit dem erfindungsgemäßen zellulären Testsystem können Sepsispatienten frühzeitig und zielgerichtet therapiert werden.

In einer bevorzugten Variante ist vorgesehen, dass die transgene Zelle zumindest zwei verschiedene TLR co-exprimiert, so dass es zur Bildung von TLR-Heterodimeren kommt, die ihre eigene Spezifität besitzen (siehe Tabelle 1). Dazu weist die Zelle bevorzugt ein Gen oder Gene, kodierend für einen ersten Toll-like Rezeptor-Typ (TLR-Typ), und zusätzlich ein Gen oder Gene, kodierend für einen zweiten Toll-like Rezeptor-Typ (TLR-Typ), auf.

Unter einem "Reportergen" wird ein oder mehrere Gene oder Genkonstrukte verstanden, die unter Kontrolle eines induzierbaren Promotors für Enzymaktivität kodieren, die im Wirtsorganismus nicht oder nur in unwesentlichem Maße konstitutiv exprimiert wird. Das Auftreten der kodierten Enzymaktivität zeigt die Induktion des Reportergenpromotors an. Vorzugsweise liegen Reportergen und indizierbarer Promotor auf einem Reportergenplasmid. Es ist vorgesehen, den Reportergenpromotor durch einen Transkriptionsfaktor zu induzieren, der, ohne an die Theorie gebunden zu sein, Bestandteil der TLR-induzierten intrazellulären Signalkaskade ist. Bevorzugt steht das erfindungsgemäß vorgesehene mindestens eine Reportergen unter der Kontrolle des Transkriptionsfaktors "nukleärer Faktor kappa-B" (NF-κB). Auf Aktivierung der TLR wird im Zytoplasma lokalisiertes gebundenes NF-κB freigesetzt und transloziert in den Zellkern. Ein bevorzugter NF-κB induzierbarer Promotor ist der Selektin oder ELAM-1 (endothelial cell leukocyte adhesion molecule-1) Promotor.

Ein bevorzugtes Reportergen ist das SEAP (sezernierte alkalische Phosphatase), vorzugsweise unter Kontrolle des ELAM-1 Promotors, vorzugsweise in Form eines Reportergenplasmids. Ein weiteres bevorzugtes Reportergen ist das beta-Galactosidasegen lacZ, vorzugsweise unter Kontrolle des ELAM-1 Promotors, vorzugsweise in Form eines Reportergenplasmids. Ein weiteres bevorzugtes Reportergen ist Luciferasegen, vorzugsweise unter Kontrolle des ELAM-1 Promotors, vorzugsweise in Form eines Reportergenplasmids. Ein weiteres bevorzugtes Reportergen ist GFP (green fluorescent protein), vorzugsweise unter Kontrolle des ELAM-1 Promotors, vorzugsweise in Form eines Reportergenplasmids. Selbstverständlich kann jeder andere für den jeweiligen Anwendungszweck geeignete Promoter eingesetzt werden, der die Eigenschaft aufweist, durch eine über die Aktivierung oder Bindung des TLR ausgelöste Signalkaskade moduliert zu werden.

Weitere TLR, die die erfindungsgemäße Zelle zusätzlich exprimieren kann, sind ausgewählt aus den zehn derzeit bekannten humanen TLRs. Es versteht sich, dass die Erfindung nicht auf die bekannten humanen TLRs beschränkt ist. Weitere noch zu benennende TLRs sind in die vorliegende Erfindung mit einbezogen. So ist ein erfindungsgemäßer Gegenstand auch eine transgene Zelle oder Zelllinie, die mindestens ein Gen einer bisher nicht näher bezeichneten TLR-Variante aufweist und diese TLR-Variante exprimiert.

In einer bevorzugten Variante der Erfindung exprimiert die Zelle oder Zelllinie zusätzlich zumindest den humanen TLR Typ 1 (TLR-1). In einer weiteren bevorzugten Variante exprimiert die Zelle oder Zelllinie zusätzlich zumindest den humanen TLR Typ 3 (TLR-3). In einer weiteren bevorzugten Variante exprimiert die Zelle oder Zelllinie zusätzlich zumindest den humanen TLR Typ 5 (TLR-5). In einer weiteren bevorzugten Variante exprimiert die Zelle oder Zelllinie zusätzlich zumindest den humanen TLR Typ 6 (TLR-6). In einer weiteren bevorzugten Variante exprimiert die Zelle oder Zelllinie zusätzlich zumindest den humanen TLR Typ 7 (TLR-7). In einer weiteren bevorzugten Variante exprimiert die Zelle oder Zelllinie zusätzlich zumindest den humanen TLR Typ 8 (TLR-8). In einer weiteren bevorzugten Variante exprimiert die Zelle oder Zelllinie zusätzlich zumindest den humanen TLR Typ 9 (TLR-9). In einer weiteren bevorzugten Variante exprimiert die Zelle oder Zelllinie zusätzlich zumindest den humanen TLR Typ 10 (TLR-10). In einer bevorzugten Variante exprimiert die Zelle oder Zelllinie zumindest den heterodimeren Rezeptor aus humanem TLR Typ 1 (TLR-1) und humanem TLR Typ 2 (TLR-2). In einer weiteren bevorzugten Variante exprimiert die Zelle oder Zelllinie zusätzlich zumindest den heterodimeren Rezeptor aus humanem TLR Typ7 (TLR-7) und humanem TLR Typ 8 (TLR-8). In einer weiteren bevorzugten Variante exprimiert die Zelle oder Zelllinie zumindest den heterodimeren Rezeptor aus humanem TLR Typ 6 (TLR-6) und humanem TLR Typ 2 (TLR-2). Die Erfindung betrifft auch die Co-Expression weiterer TLR, ausgewählt aus der Gruppe bestehend aus TLR-1, TLR-3, TLR-5, TLR-6, TLR-7, TLR-8, TLR-9 und TLR-10. Bevorzugt betrifft die Erfindung also folgende Heterodimere: TLR-1/TLR-2; zusätzlich TLR1-TLR-3; TLR-1/TLR-4; zusätzlich TLR-1/TLR-5; zusätzlich TLR-1/TLR-6; zusätzlich TLR-1/TLR-7; zusätzlich TLR-1/TLR-8; zusätzlich TLR-1/TLR-9; zusätzlich TLR-1/TLR-10; TLR-2/TLR-3; TLR-2/TLR-4; TLR-2/TLR-5; TLR-2/TLR-6; TLR-2/TLR-7; TLR-2/TLR-8; TLR-2/TLR-9; TLR-2/TLR-10; TLR-3/TLR-4; zusätzlich TLR-3/TLR-5; zusätzlich TLR-3/TLR-6; zusätzlich TLR-3/TLR-7; zusätzlich TLR-3/TLR-8; zusätzlich TLR-3/TLR-9; zusätzlich TLR-3/TLR-10; TLR-4/TLR-5; TLR-4/TLR-6; TLR-4/TLR-7; TLR-4/TLR-8; TLR-4/TLR-9; TLR-4/TLR-10; zusätzlich TLR-5/TLR-6; zusätzlich TLR-5/TLR-7; zusätzlich TLR-5/TLR-8; zusätzlich TLR-5/TLR-9; zusätzlich TLR-5/TLR-10; zusätzlich TLR-6/TLR-7; zusätzlich TLR-6/TLR-8; zusätzlich TLR-6/TLR-9; zusätzlich TLR-6/TLR-10; zusätzlich TLR-7/TLR-8; zusätzlich TLR-7/TLR-9; zusätzlich TLR-7/TLR-10; zusätzlich TLR-8/TLR-9; zusätzlich TLR-8/TLR-10; zusätzlich TLR-9/TLR-10. Diese können zusätzlich jeweils allein oder in Kombination zumindest einem mit anderen TLR oder TLR-Heterodimer co-exprimiert werden.

In einer bevorzugten Ausführung der Erfindung wird nicht nur die einzige erfindungsgemäße transgene Zelle oder Zelllinie bereitgestellt. Bevorzugt ist ein "Set" aus mindestens zwei verschiedenen erfindungsgemäßen Zelltypen, die jeweils unterschiedliche TLRs oder TLR-Heterodimere exprimieren. Besonders bevorzugt sind "Sets" aus drei, vier, fünf, sechs, sieben, acht, neun, zehn oder mehr verschiedenen erfindungsgemäßen Zelltypen, die jeweils unterschiedliche TLRs oder TLR-Heterodimere exprimieren. Ohne an die Theorie gebunden zu sein, binden einzelne Pyrogene einer Pyrogenpopulation jeweils spezifisch an ein oder wenige bestimmte TLRs und/oder TLR-Heterodimere. Auf diese Weise ist eine einfache Charakterisierung einzelner Pyrogene und/oder des Pyrogenspektrums einer Pro be möglich. Beispielsweise erlaubt ein Zelltyp, der das Heterodimer aus TLR-2 und TLR-6 exprimiert, den spezifischen Nachweis von Mycoplasma-Pyrogenen und Hefe-Pyrogenen. Beispielsweise erlaubt ein "Set" aus einem Zelltyp, der zusätzlich TLR-3 exprimiert, und einem Zelltyp, der zusätzlich TLR-9 exprimiert, den spezifischen Nachweis von Viren mit doppelsträngiger RNA.

Ein Gegenstand der Erfindung ist deshalb auch ein Zellkulturgefäß, vorzugsweise eine Zellkulturplatte, Multiwellplatte, worin mindestens ein Zelltyp, bevorzugt mehrere verschiedene Zelltypen eingebracht, adhäriert, oder als Suspension inkubiert sind. Im einfachsten Fall liegen die Zellen an der Oberfläche des Zellkulturgefäßes, z.B. an Collagenfilm, adhäriert vor. Bevorzugt ist aber auch die Kultivierung in Suspension. Inkubation/Kultivierung auf oder in 3D-Biomatrices ist ebenfalls möglich. Weiter ist es möglich, jeweils verschiedene Zelltypen in adressierbaren Subkompartimentierungen eines Zellkulturträgers auf-/oder einzubringen. Die Erfindung sieht vor, die Zellen auf die Platten, Gefäße oder Wells, auszusäen und für die weitere Verwendung zu Lagern, vorzugsweise einzufrieren, zu kryokonservieren. So vorbereitete Platten, Gefäße oder Wells können dann bei Bedarf innerhalb kurzer Zeit für entsprechende Tests auf Pyrogene und andere TLR aktivierende oder modulierende Wirkstoffe (TLR-Antagonisten) eingesetzt werden. Im einfachsten Fall werden die Platten etc. mit den Zellen aufgetaut, mit der zu testenden Probe inkubiert. Die Reportergen vermittelte Enzymaktivität wird in an sich bekannter Weise nachgewiesen und zeigt in dem Zelltyp das Vorhandensein oder die Abwesenheit einer spezifischen Aktivierung des TLR durch die zu testende Substanz, Pyrogen oder PAMP an.

Das Zellkulturgefäß wird bevorzugt in einem Kit bereitgestellt. Das Kit enthält die vorstehend charakterisierenden Zellen in einem vorstehend beschriebenen Zellkulturgefäß oder Assayträger und wird vorzugsweise im eingefrorenen Zustand vor allem zur sofortigen Durchführung des Tests bereitgestellt. Bei der Verwendung des Kits erübrigt es sich vorteilhafterweise, aufwändige Zellkulturbedingungen wie einen CO₂-Brutschrank einzusetzen. Das Kit kann beispielsweise in einem einfachen Labor eines Krankenhauses mit Mitteln zur Detektion des Farbumschlags durchgeführt werden. Im einfachsten Fall genügt bereits der augenscheinlich erkennbare Farbumschlag die spezifische TLR-Aktivierung an. Aus dem Muster des Farbumschlags kann der Verwender des Kits auf das Pyrogen-spektrum und/oder den Erregertyp schließen.

Ein erfindungsgemäßes Kit zum spezifischen Nachweis eines Pyrogens in einer Probe, enthält mindestens eine transgene Zelle nach einem der vorstehenden Ansprüche in einem Kulturgefäß und vorzugsweise Detektionsmedium, enthaltend zumindest ein Substrat für das von dem induzierbaren Reportergen kodierte Enzym. Bevorzugt ist ein Kit, das ein Zellkulturgefäß oder- platte mit mindestens zwei Kompartimenten oder Wells enthält, wobei in einem ersten Well mindestens eine erste transgene Zelle, exprimierend mindestens einen ersten TLR-Typ oder Heterodimer, und in einem zweiten Well ein von der ersten transgenen Zelle verschiedenen zweite transgene Zelle, exprimierend mindestens einen zweiten TLR-Typ oder Heterodimer, enthalten ist.

Demgemäß sieht eine besonders bevorzugte Ausführung der Erfindung vor: Ein Kit, bestehend aus einem oder mehreren Zellkulturgefäßen mit einem Well, enthaltend die transgene Zelle nach Anspruch 1 und bevozugt einen ersten weiteren Well, enthaltend eine erste weitere transgene Zelle, vorzugsweise exprimierend zumindest den humanen TLR-1, einem zweiten weiteren Well, enthaltend eine zweite weitere transgene Zelle, vorzugsweise exprimierend zumindest den humanen TLR-3, bevorzugt einem dritten weiteren Well, enthaltend eine dritte weitere transgene Zelle, vorzugsweise exprimierend zumindest den humanen TLR-5, bevorzugt einem vierten weiteren Well, enthaltend eine vierte weitere transgene Zelle, vorzugsweise exprimierend zumindest den humanen TLR-6, bevorzugt einem fünften weiteren Well, enthaltend eine fünfte weitere transgene Zelle, vorzugsweise exprimierend zumindest den humanen TLR-7, bevorzugt einem sechsten weiteren Well, enthaltend eine sechste weitere transgene Zelle, vorzugsweise exprimierend zumindest den humanen TLR-8, bevorzugt einem siebten weiteren Well, enthaltend eine siebte weitere transgene Zelle, vorzugsweise exprimierend zumindest den humanen TLR-9, und bevorzugt einem achten weiteren Well, enthaltend eine achte weitere transgene Zelle, vorzugsweise exprimierend zumindest den humanen TLR-10, sowie bevorzugt Detektionsmedium.

Ein weiterer Gegenstand der Erfindung ist auch ein Verfahren zum spezifischen Nachweis eines Pyrogens in einer Probe. Erfindungsgemäß umfasst das Verfahren zumindest die Schritte: Bereitstellen einer Probe, Bereitstellen mindestens einer erfindungsgemäßen transgenen Zelle oder Zelllinie, mindestens einen spezifischen TLR oder ein spezifisches TLR-Heterodimer exprimiert; Inkontaktbringen der Probe mit der Zelle, wodurch ein so genannter "Probe-Zelle-Komplex" entsteht, der besonders durch die Bindung von Probenbestanteilen an die Zelle charakterisiert ist; Inkubieren des Probe-Zelle-Komplexes zur Induktion der Enzymaktivität, vorzugsweise bei etwa 37 °C für etwa 3 bis etwa 24 Std. und Detektion oder Nachweis der von dem Reportergen induzierten Enzymaktivität, wobei die Enzymaktivität das Vorhandensein eines für den TLR-Typ oder TLR-Heterodimer der Zelle oder Zelllinie spezifischen Pyrogens oder agonistischen Wirkstoff anzeigt.

Vorzugsweise findet die Detektion oder Nachweis der von dem Reportergen induzierten Enzymaktivität, durch Bereitstellen eines Detektionsmediums, enthaltend Substrat für das von dem induzierbaren Reportergen der Zelle kodierte Enzym, und durch Inkubation des induzierten Probe-Zelle-Komplexes im Detektionsmedium, vorzugsweise bei etwa 37 °C und vorzugsweise für etwa 30 bis etwa 240 min, statt, wobei die Enzymaktivität durch Nachweis des enzymatisch umgesetzten Substrats detektiert und bevorzugt quantifiziert wir. Die Quantifizierung des enzymatisch umgesetzten Substrats und damit der Enzymaktivität erlaubt Rückschlüsse auf die Aktivität und Konzentration des spezifischen Pyrogens oder TLR aktivierenden Wirkstoffs (TLR-Agonist; CpG-Motiv etc.).

Bevorzugt ist die Enzymaktivität eine alkalische Phosphatase-Aktivität, die vorzugsweise durch die SEAP vermittelt wird. Alkalische Phosphatasen sind Enzyme, die im alkalischen Milieu die Hydrolyse von Phosphorsäure-Estern katalysieren. Als Substrat wird bevorzugt 5-bromo-4-chloro-Indoylphosphat (BCIP) eingesetzt. Die Detektion der Enzymaktivität erfolgt dabei durch blauen Farbumschlag und/oder blaues Präzipitat, ein tiefblau gefärbter, unlöslicher und leicht erkennbarer Niederschlag von Indigo.

In einer alternativen Variante ist das Substrat p-Nitrophenylphosphat (pNPP) ist und die alkalische Phosphatase-Aktivität wird durch hydrolytische Spaltung des pNPP durch gelben Farbumschlag der Lösung angezeigt. Der gelbe Farbumschlag der Lösung wird vorzugsweise photometrisch detektiert und quantifiziert. Bevorzugt findet die photometrische Analyse bei etwa 405 nm statt. Aus der Extinktion lässt sich die Konzentration des Pyrogens oder TLR aktivierenden Wirkstoffs in der Probe bestimmen. Um Reportergene, die intrazellulär nachgewiesen werden, auch quantifizieren zu können, müssen die Zellen lysiert und der Farbstoff daraus freigesetzt werden. Ein direkter intrazellulärer Nachweis erfolgt durch Lösen des Farbstoffes in den Zellen über NaOH; dadurch sind intrazelluläre quantitative Messungen möglich. Selbstverständlich ist zur Quantifizierung auch eine densitometrische Auswertung (über Graustufen) möglich.

In einer anderen bevorzugten Variante ist die Enzymaktivität eine beta-Galaktosidase-Aktivität und das Substrat vorzugsweise 5-bromo-4-chloro-3-indoyl-beta-D-Galaktopyranosid (X-Gal). Die Detektion der Enzymaktivität erfolgt durch blauen Farbumschlag und/oder blaues Präzipitat.

In einer anderen bevorzugten Variante ist die Enzymaktivität eine Luciferase-Aktivität und das Substrat vorzugsweise Luciferin. In Gegenwart von gegebenenfalls zusätzlich zugegebenem ATP und Mg²⁺ wird die Enzymaktivität durch Lumineszenz angezeigt (Chemilumineszenzassay).

Die Probe, die mittels des erfindungsgemäßen Verfahrens bzw. Testsystems analysiert werden kann, ist besonders eine klinische Probe aus einem menschlichen oder tierischen Körper. Vorzugsweise ist die Probe Blut, vorzugsweise Vollblut, beispielsweise im Falle der Sepsis. Weitere klinische Proben sind Blutserum, Blutplasma, Urin, Sputum, Stuhl, Gewebebiopsie, Bronchiallavage, ZNS-Liquor, Rückenmarksliquor, Lymphe, Synovialflüssigkeit und ähnliche, beispielsweise zur Typisierung von Infektionen Ein weiterer Gegenstand der Erfindung ist daher die Verwendung der transgenen Zelle zum spezifischen Nachweis eines Pyrogens in einer klinischen Probe, vorzugsweise nach dem erfindungsgemäßen Verfahren und/oder vorzugsweise unter Verwendung des erfindungsgemäßen Kits.

Es hat sich überraschend gezeigt, dass die erfindungsgemäße transgene Zelle oder Zelllinie einem Testsystem eingesetzt werden kann, um Erzeugnisse auf Pyrogenfreiheit zu testen. Wird das Verfahren zur Prüfung auf Pyrogenfreiheit oder zur Bestimmung der Pyrogenkontamination eingesetzt, ist die Probe vorzugsweise ein Prüfstück (Specimen) eines medizinischen Instruments oder Medizinprodukts (MP) oder in vitro Diagnostikums (IVD) oder ein Arzneimittel, Arzneimittelbestandteir, Nahrungsmittel, Nahrungsmittelbestandteil oder Roh- oder Ausgangsstoff für Nahrungsmittel oder Arzneimittel. Darunter zählen chirurgische Instrumente, Kanülen, Spritzen, Infusionsbestecke, Blutbeutel und Transfusionsbestecke, Dialysebestecke und -apparaturen, Wundauflagen, Nahtmaterial, Implantate, Prothesen, Katheder, Infusionslösungen, Spüllösungen und ähnliche. Weiter ist vorgesehen, dass die Probe ausgewählt ist aus Transplantaten, Geweben und Zellen menschlichen Ursprungs und Produkten dieses Inhalts oder dieses Ursprungs sowie Transplantaten, Geweben, Zellen tierischen Ursprungs und Produkten dieses Inhalts oder dieses Ursprungs. Weiter ist vorgesehen, dass die Probe ausgewählt ist aus kosmetischen Artikeln und Kosmetik. Ein weiterer Gegenstand der Erfindung ist daher die Verwendung der transgenen Zelle zum Testen von solchen Erzeugnissen auf Pyrogenfreiheit, vorzugsweise nach dem erfindungsgemäßen Verfahren und/oder vorzugsweise unter Verwendung des erfindungsgemäßen Kits.

Weiter hat sich überraschend gezeigt, dass die erfindungsgemäße transgene Zelle oder Zelllinie einem Testsystem eingesetzt werden kann, um Wirkstoffe mit der Eigenschaft eines TLR-Antagonisten in einer Gruppe von Kandidatensubstanzen aufzufinden und ihre Wirksamkeit zu quantifizieren. Ein weiterer Gegenstand der Erfindung ist daher auch die Verwendung der transgenen Zelle zum Screenen von Wirkstoffen mit der Eigenschaft eines TLR Antagonisten, vorzugsweise nach dem erfindungsgemäßen Verfahren und/oder vorzugsweise unter Verwendung des erfindungsgemäßen Kits.

Schließlich hat sich überraschend gezeigt, dass die erfindungsgemäße transgene Zelle oder Zelllinie einem Testsystem eingesetzt werden kann; um Oligonucleotiden mit CpG-Motiven, die bestimmte TLR, besonders TLR-9, aktivieren, einer Gruppe von Kandidatensubstanzen aufzufinden und ihre Wirksamkeit zu quantifizieren. Ein weiterer Gegenstand der Erfindung ist daher schließlich auch Verwendung der transgenen Zelle zum Screenen von Oligonucleotiden mit CpG-Motiven, vorzugsweise nach dem erfindungsgemäßen Verfahren und/oder vorzugsweise unter Verwendung des erfindungsgemäßen Kits.

### Ausführungsbeispiele

Es zeigen:

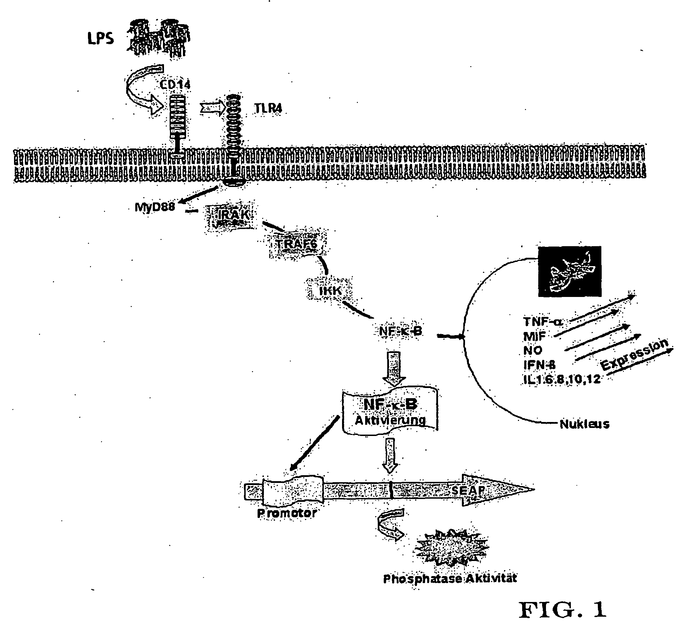

Figur 1: Schematische Darstellung des erfindungsgemäßen Testverfahrens (am Beispiel von TLR4/CD14 (MD2) mit dem Liganden LPS)
Figur 2: NIH-3T3 Klon 4/5 TLR-4/CD14 mit SEAP-Reporterplasmid nach dem Auftauen und nach Zugabe von 100 ng/ml LPS (4-well rechte Seite) und Detektionsmedium mit BCIP-Substrat
Figur 3: Induktion des NIH-3T3 TLR-4/CD14 Testsystems mit 10 pg/ml bis 100 pg/ml LPS; Substrat: BCIP; Negativkontrolle wurde nicht induziert oder mit ssRNA40 induziert.
Figur 4: Sensitivitätsnachweis des NIH-3T3 Klon 4/5 TLR-4/CD14; LPS spezifisch bis 10 pg/ml, 2 Stunden nach Zugabe des Detektionsmediums: photometrische Analyse
Figur 5: Sensitivitätsnachweis des NIH-3T3 Klon 4(5) TLR-4/CD14; LPS ist spezifisch bis 1 pg/ml nachweisbar, 2 Stunden nach Zugabe des Detektionsmediums: photometrische Analyse
Figuren 6 und 7: Vergleichsexperiment: TLR-4 Test mit HEK blue 293 Fibroblasten und andere 293 Fibroblasten, transfiziert mit TLR-4/CD14 SEAP, induziert mit 100 ng/ml LPS; sowohl die induzierte, als auch die nicht induzierte Kontrolle zeigt einen blauen Farbumschlag; ein spezifischer Nachweis ist mit diesen Zellen nicht möglich.
Figur 8: Vergleichsexperiment: TLR-4 Test mit HEK blue 293 Fibroblasten und anderen 293 Fibroblasten, transfiziert mit TLR-4/CD14 SEAP, induziert mit 100 ng/ml LPS: SDS-PAGE/Western Blot Analyse der Zellpellets, primäre Antikörper: Anti-SEAP sowie Anti-Maus POD konjugierte sekundäre Antikörper, Marker: SeeBlue® Plus2 pre-stained, Standard: alkalische Phosphatase (SAEP); in HEK293 und anderen 293 Zellen (K2 und K4) wird sowohl in den induzierten Zellen, als auch in den nicht induzierten Kontrollzellen in gleicher Menge exprimiert; ein spezifischer Nachweis ist mit diesen Zellen nicht möglich
Figur 9: Spezifität des Testsystems: NIH-3T3 TLR-4/CD14 Testsystem wurde mit unspezifischen Pyrogenen induziert (jeweils 25 µg/ml); ODN (Ligand für TLR-9), PGN (Ligand für TLR-2), Poly IC; es findet kein Farbumschlag statt, der Test reagiert spezifisch.
Figur 10: Phasenkontrastaufnahme des NIH-3T3 TLR-4/CD14 Klon 4/5: 30.000 Zellen/ Well ausgesät, 100 µl in 30% FCS, 80 mmol/l HEPES und 5% DMSO, eingefroren für 3 Tage bis 4 Wochen bei 80 °C; Adhäsion über Nacht 37°C, humide Atmosphäre ohne CO₂
Figur 11: Erfindungsgemäßer TLR-4 Test, durchgeführt an eingefrorenen und wieder aufgetauten NIH-3T3 TLR-4/CD14 SEAP P40, induziert mit 100 pg/ml LPS und 100 pg/ml ssRNA40, Induktion nach 24 Std., Detektion nach 3 Std.; eine spezifische Blaufärbung der induzierten Zellen ist zu beobachten.
Figur 12: Erfindungsgemäßer TLR-5 Test, durchgeführt an NIH-3T3 Klone TLR-5 SEAP, induziert mit 2 µg/ml Flagellin; eine spezifi-sche Blaufärbung der induzierten Zellen ist zu beobachten.
Figur 13: Vergleichsexperiment: TLR-5 Test mit HELA-Zellen, transfiziert mit TLR-5 SEAP, induziert mit 2 µg/ml Flagellin; sowohl die induzierten Zellen, als auch die nicht induzierte Kontrolle zeigen intensive Blaufärbung. Mit dieser Zelllinie ist keine spezifische Induktion möglich.

### Beispiel 1: TLR-4 spezifisches Testsystem

### Methoden

### Transfektion mit TLR-4

Die Zelllinie NIH-3T3 wurde mit einem TLR4/CD14-Komplex sowie dem Reportergenplasmid SEAP/ELAM-1 transfiziert.

Die Endotoxin (LPS)-vermittelte Induktion des TLR-4 führt nach einer Signalkaskade zu der Aktivierung des Transkriptionsfaktors NF-κB.

Die Expression des Reportergens SEAP wird von einem NF-κB induzierbaren ELAM-1 Promotor kontrolliert. Damit erfolgt auf eine Induktion des TLR-4 durch das Endotoxin Lipopolysaccharid, LPS die NF-κB Aktivierung und damit spezifisch die Sezernierung der SEAP.

### Durchführung des Tests

NIH-3T3 TLR-4/CD14 Klon 4/5 P35 werden in einer Dichte von 30.000 bis 200.000 Zellen/weil (24-well) (entsprechende Zellzahl für andere well-Volumina) in 500 µl/well ausgesät in 0,5% FCS Medium o/n zum Adhärieren;

Am folgenden Tag erfolgt die Induktion mit LPS o/n (+ Negativkontrolle: ssRNA40 kann nicht von TLR-4/CD14 detektiert werden)

An folgenden Tag erfolgt die Detektion durch Inkubation mit 300 µl/well Detektionsmedium, welches den induzierten Zellen direkt zugegeben wird: In den induzierten Zellen setzt SEAP-Aktivität das Substrat BCIP im Detektionsmedium in ein tiefblaues, unlösliches Endprodukt (Indigo) um. Alternativ setzt in den induzierten Zellen SEAP-Aktivität das Substrat pNPP im Detektionsmedium in einen hellgelben löslichen Farbkomplex um, der bei etwa 405 nm photometrisch bestimmt wird. Die photometrische Analyse erfolgt etwa 2 Stunden nach Zugabe des Detektionsmediums

### Einfrieren und Anwendung des Testkits mit 24-well Platte

In einer Variante werden transfizierten Zelllinien im entsprechenden Assayformat, hier: eine Zellkultur-Well-Platte (Multiwellplate), in entsprechender Dichte (200.000 Zellen/well in je 500 µl (bei 24-well)) eingefroren.

Der Anwender bekommt den "Assay-Kit" mit 8-10 verschiedenen Zelllinien im entsprechenden Medium bereits in der Testplatte auf Trockeneis gekühlt geliefert. Der Assay kann der Verpackung entnommen und direkt in einem 37°C Schrank inkubiert werden.

Nach dem Auftauen des Kits erfolgt die Zugabe von DMEM Kulturmedium zur Adhäsion der Zellen über Nacht. Ein Medium mit HEPES-Puffer ermöglicht eine Inkubation des Zell-Tests in einem Wärmeschrank d.h. ohne CO₂-Begasung. Induziert wird testweise durch Zugabe von 100 ng/ml LPS. Die Detektion wird nach 24 Stunden durch Zusatz von 300 µl/well BCIP-Detektionsmedium durchgeführt.

### Ergebnisse

### a) aufgetautes Testsystem

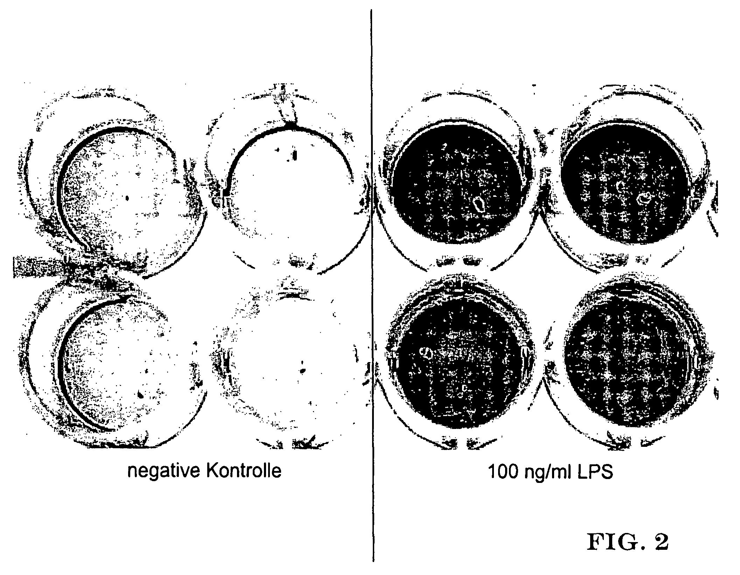

Figur 2 zeigt die Ergebnisse bei negativer Kontrolle und bei 100 ng/ml LPS an dem erfindungsgemäßen 3T3 NIH Klon 4/5 TLR-4/CD14 nach dem Auftauen. In den induzierten Zellen setzt SEAP-Aktivität das Substrat BCIP im Detektionsmedium in ein tiefblaues, unlösliches Endprodukt (Indigo) um.

Es wurde ein schnelles, einfaches, ohne großen apparativen Aufwand und Zellkulturlabor (Sterilbank und CO₂-Brutschrank etc.) zu betreibendes Nachweissystem für LPS auf der Basis von Zellen, die mit TLR 4/CD14 stabil transfiziert wurden, entwickelt. Es ist schnell durchführbar und einfach in der Handhabung.

### b) Sensitivität

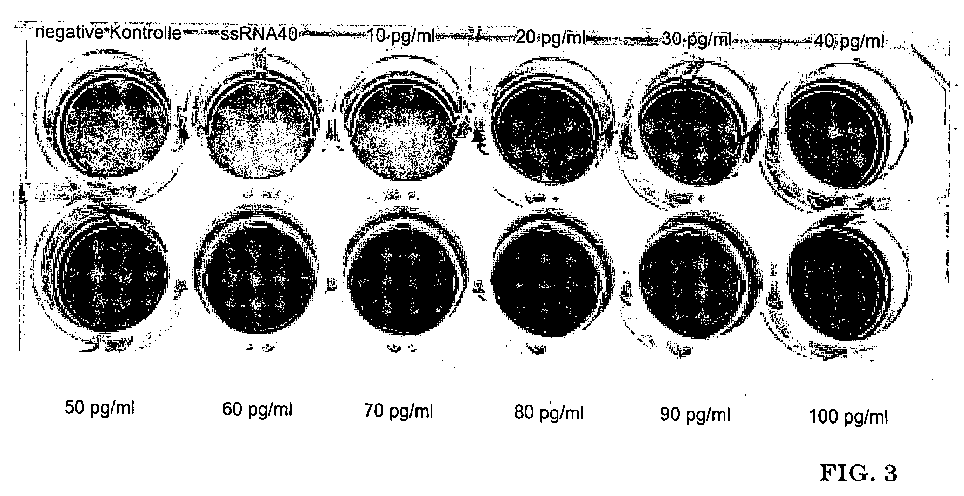

Figur 3 zeigt die Induktion des NIH-3T3 Klon 4(5) TLR4/CD14 SEAP Testsystems mit 10 µg/ml bis 100 pg/ml LPS. Substrat des Detektionsmediums ist BCIP. Eine Negativkontrolle wurde nicht induziert, eine andere Negativkontrolle wurde mit ssRNA40 unspezifisch induziert.

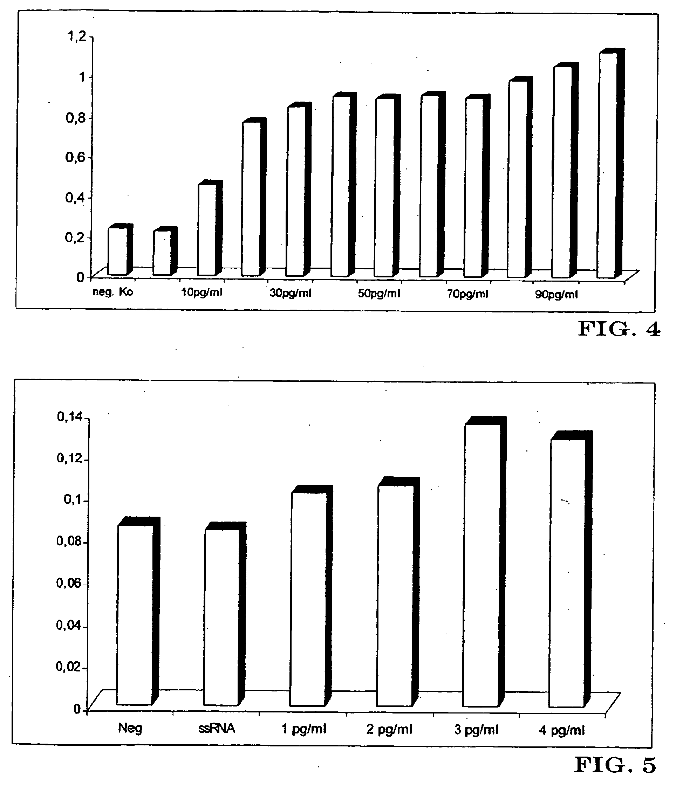

Figur 4 zeigt den Sensitivitätsnachweis des NIH-3T3 Klon 4(5) TLR4/CD14 SEAP spezifisch bis 10 pg/ml LPS. Figur 5 zeigt den Sensitivitätsnachweis des NIH-3T3 Klon 4(5) TLR4/CD14 SEAP spezifisch bis 1 pg/ml LPS.

Die Sensitivität des Testsystems liegt bei etwa 1 bis 2 pg/ml LPS.

### c) Spezifität

Das vorgenannte NIH-3T3 TLR-4/CD14 SEAP Testsystem wurde mit hohen Mengen unspezifischen Pyrogenen (jeweils 25 µg/ml ODN, PGN, Poly IC) induziert für die TLR-4 nicht bindet: ODN, PGN, Poly IC werden von TLR 9, 2 und 3 nicht aber von TLR-4 erkannt. Figur 5 zeigt das Ergebnis: Auch bei extrem hohem unspezifischem Pyrogenanteil ist kein Farbumschlag im TLR-4 spezifischen System zu sehen; der TLR-4 Test ist spezifisch.

### Beispiel 2: Vergleichsexperimente

### Methode

HEK blue 293 Fibroblasten und andere 293 Fibroblasten wurden mit TLR-4/CD14 SEAP transfiziert und mit 100 ng/ml LPS induziert. Alle weiteren Verfahrensparameter wurden wie im erfindungsgemäßen Beispiel 1 gewählt.

Zusätzlich wurde eine Western-Blot Analyse der Genexpression in an sich bekannter Weise durchgeführt: Erstantikörper: Anti-SEAP; konjugierter Zweitantikörper: Anti-Maus POD; Marker: SeeBlue® Plus2; Pre-Stained Standard.

### Ergebnisse

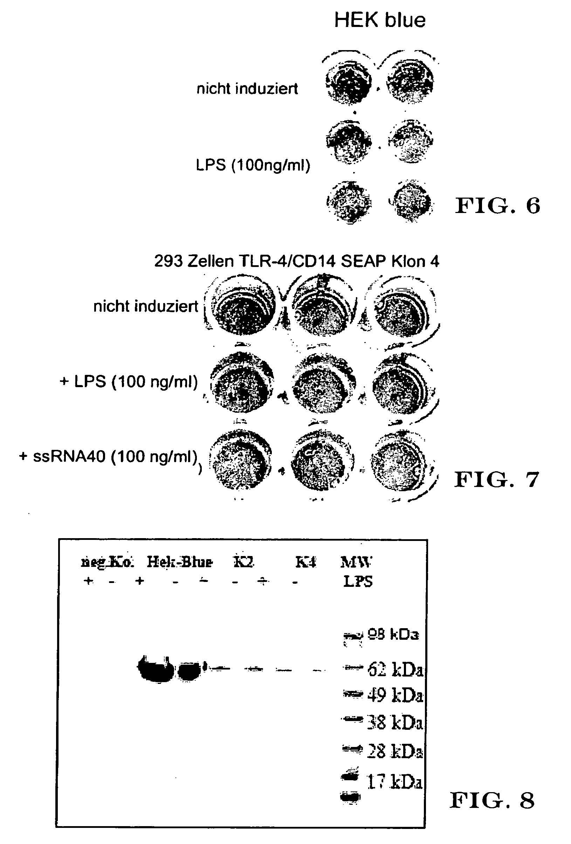

Die Figuren 6 und 7 zeigen die Ergebnisse des Farbtests: Nicht nur die induzierten Zellen, sondern auch die nicht induzierten Kontrollen zeigen einen blauen Farbumschlag. Mit den HEK blue 293 und anderen 293 Zellen wie Klon 4(K4) ist keine spezifische Induktion und damit keine Etablierung des Testsystems möglich.

Die Figur 8 zeigt die Ergebnisse der SDS-PAGE/Western Blot Analyse der Zellpellets nach Induktion mit 100 ng/ml LPS: Die Alkalische Phosphatase wird in den HEK293 und in anderen 293 Zellen (K2 und K4), in den nicht induzierten Kontrollzellen in gleicher Menge wie in den induzierten Zellen exprimiert; ein spezifischer Nachweis von TLR aktivierenden Wirkstoffen, Pyrogenen, PAMPs ist mit diesen Zellen nicht möglich.

### Beispiel 3: Assay im 96-well Maßstab und CO₂ freier Kultivierung:

### Methode

Auf einer Multiwellplate (96-well) wurden NIH 3T3 TLR4 CD14 SEAP P40 Zellen in einer Dichte von 30.000 Zellen/weil in 100 µl Medium (DMEM 80mmol/l HEPES, 30% FCS und 5% DMSO) in Suspension bei -80°C eingefroren.

Nach 72 Std. bis 4 Wochen bei -80 °C wurden die Zellen durch Zugabe von 100 µl Medium (10% FCS) bei 37°C CO₂-frei aufgetaut.

Nach 24 Std. Adhäsion wurde auf Medium (DMEM 0,5% FCS) gewechselt und die Zellen in 100 µl mit 30 pg/ml LPS oder 30 pg/ml ssRNA33 (Kontrolle) induziert.

Nach 24 Std. Induktion wurde ein Medienwechsel auf Detektionsmedium durchgeführt. Alternativ wird 100 µl/well Detektionsmedium nach Induktionszeit ins Well direkt zugegeben.

### Ergebnisse

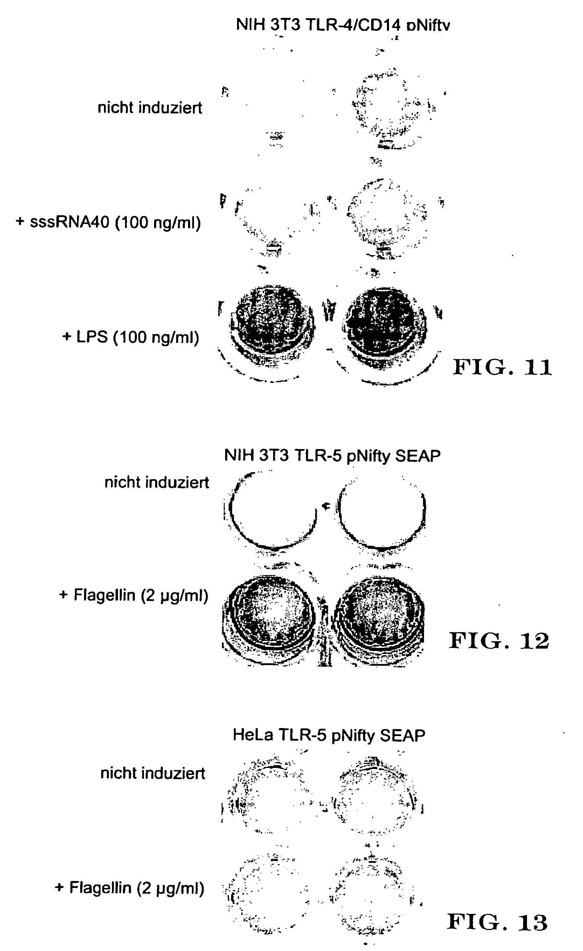

Figur 11 zeigt die Ergebnisse: Nach spätestens 3 bis 24 Std. ist die Detektion der induzierten Enzymaktivität eindeutig. Wird Detektionsmediums nach Induktionszeit direkt ins Well gegeben, ist nach 1 bis 3 Std. ist ein Signal nachweisbar.

### Beispiel 4: Histologie bei CO₂ freier Kultivierung

### Methode

Auf einer Multiwellplate wurden NIH 3T3 TLR4 CD14 SEAP Zellen in einer Dichte von 30.000 Zellen/well in 100 µl Medium (30% FCS, 80 mmol/l HEPES) ausgesät. Die Adhäsion erfolgte über Nacht bei 37°C in humider Atmosphäre ohne CO₂

### Ergebnisse

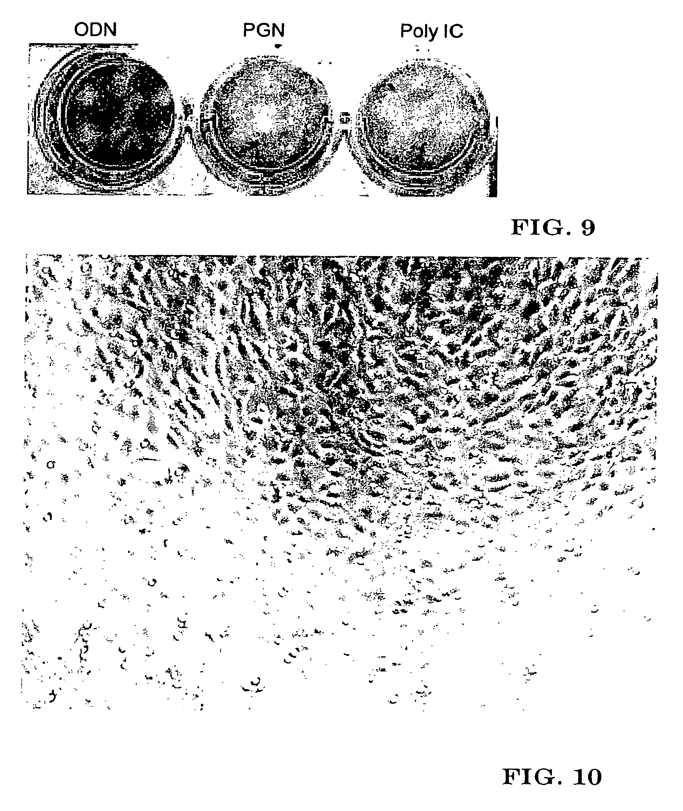

Figur 10 zeigt die Phasenkontrastaufnahme der adhärierten Zellen im Well: Die Zellen wachsen bei einer Kultivierung im 37°C Wärmeschrank in einem HEPES gepufferten Medium. Das Bild zeigt einen intakten Zellmonolayer.

### Beispiel 5: TLR-5 spezifisches Testsystem

### Methode

### Transfektion mit TLR-5

Die Zelllinie NIH-3T3 wurde mit TLR-5 sowie dem Reportergenplasmid SEAP transfiziert. Die Maßnahmen entsprechen Beispiel 1.

### Durchführung des Tests

NIH-3T3 Klon TLR-5 werden in einer Dichte von 30.000 bis 200.000 Zellen/well (24-well) in 500 µl/well in 0,5% FCS Medium ausgesät; die Induktion erfolgt mit 2 µg/ml Flagellin; die Detektion durch Inkubation mit 300 µl/well Detektionsmedium mit BCIP.

### Ergebnisse

Figur 12 zeigt die Ergebnisse des Farbtests: Es tritt eine spezifische Blaufärbung der induzierten Zellen auf; nicht-induzierte Kontrollzellen zeigen keine Blaufärbung.

## Patentansprüche

1. Transgene Zelle zum spezifischen Nachweis eines Pyrogens in einer Probe, enthaltend im Genom:
a) ein Gen oder Gene, kodierend für mindestens einen humanen Toll-like Rezeptor (TLR), zumindest für humanen TLR-2 und/oder humanen TLR-4, den die Zelle exprimiert, und
b) mindestens ein Reportergen, das unter der Expressionskontrolle eines durch NF-κB induzierbaren Promotors steht,
wobei die Zelle eine murine Fibroblastenzelle vom Typ NIH-3T3 ist.

2. Zelle nach einem der vorstehenden Ansprüche, wobei das Reportergen eine sezernierte alkalische Phosphatase (SEAP) kodiert.

3. Zelle nach einem der vorstehenden Ansprüche, wobei das Reportergen eine beta-Galactosidase kodiert.

4. Zelle nach einem der vorstehenden Ansprüche, wobei das Reportergen eine Luciferase kodiert.

5. Zelle nach einem der vorstehenden Ansprüche, wobei das Reportergen GFP kodiert.

6. Zelle nach einem der vorstehenden Ansprüche, wobei der induzierbare Promotor der Promotor für Selektin (endothelial cell leukocyte adhesion molecule; ELAM-1) ist.

7. Zelle nach einem der vorstehenden Ansprüche, die zusätzlich den Corezeptor Typ CD14 (MD2) exprimiert.

8. Kit zum spezifischen Nachweis eines Pyrogens in einer Probe, enthaltend: Kulturgefäß mit mindestens einer transgenen Zelle nach einem der vorstehenden Ansprüche.

9. Kit nach Anspruch 8, enthaltend: Detektionsmedium, enthaltend Substrat für das von dem induzierbaren Reportergen kodierte Enzym.

10. Kit nach Anspruch 8 oder 9, enthaltend: Zellkulturgefäß oderplatte mit mindestens zwei Kompartimenten oder Wells, wobei in einem ersten Well die mindestens eine transgene Zelle und in einem zweiten Well eine von der transgenen Zelle verschiedene andere transgene Zelle, exprimierend mindestens einen anderen TLR-Typ, enthalten ist.

11. Verfahren zum spezifischen Nachweis eines Pyrogens in einer Probe, umfassend die Schritte:
a) Bereitstellen der Probe und mindestens einer transgenen Zelle nach einem der Ansprüche 1 bis 7;
b) Inkontaktbringen der Probe mit der Zelle;
c) Inkubieren des Probe-Zelle-Komplexes zur Induktion; und
d) Detektion der von dem induzierten Reportergen vermittelten Enzymaktivität,
wobei die Detektion der Enzymaktivität das Vorhandensein eines dafür spezifischen Pyrogens anzeigt.

12. Verfahren nach Anspruch 11, wobei Schritt d) die Schritte umfasst:
d1) Bereitstellen eines Detektionsmediums, enthaltend Substrat für das von dem induzierbaren Reportergen der Zelle kodierte Enzym; und
d2) Inkubieren des induzierten Probe-Zelle-Komplexes im Detektionsmedium zur Detektion der von dem induzierten Reportergen vermittelten Enzymaktivität.

13. Verfahren nach Anspruch 11 oder 12, wobei die Enzymaktivität eine alkalische Phosphatase-Aktivität ist.

14. Verfahren nach Anspruch 13, wobei das Substrat 5-bromo-4-chloro-Indoylphosphat (BCIP) ist und die Detektion durch blauen Farbumschlag und/oder blaues Präzipitat angezeigt wird.

15. Verfahren nach Anspruch 14, wobei das Substrat p-Nitrophenylphosphat (pNPP) ist und die Detektion durch gelben Farbumschlag der Lösung angezeigt wird.

16. Verfahren nach Anspruch 15, wobei der gelben Farbumschlag der Lösung photometrisch quantifiziert wird.

17. Verfahren nach Anspruch 11 oder 12, wobei die Enzymaktivität eine beta-Galaktosidase-Aktivität ist.

18. Verfahren nach Anspruch 17, wobei das Substrat 5-bromo-4-chloro-3-indoyl-beta-D-Galaktopyranosid (X-Gal) ist und die Detektion durch blauen Farbumschlag und/oder blaues Präzipitat angezeigt wird.

19. Verfahren nach Anspruch 11 oder 12, wobei die Enzymaktivität eine Luciferase-Aktivität ist.

20. Verfahren nach Anspruch 19, wobei das Substrat Luciferin ist, gegebenenfalls zusätzlich mit ATP und Mg²⁺, und die Detektion durch Lumineszenz angezeigt wird.

21. Verfahren nach Anspruch 11 oder 12, wobei die Enzymaktivität GFP ist.

22. Verfahren nach einem der Ansprüche 11 bis 21, wobei die Probe eine klinische Probe aus einem menschlichen oder tierischen Körper ist.

23. Verfahren nach Anspruch 22, wobei die Probe Blut ist.

24. Verfahren nach einem der Ansprüche 11 bis 21, wobei die Probe ein Prüfstück eines medizinischen Instruments oder Medizinprodukts ist.

25. Verfahren nach einem der Ansprüche 11 bis 21, wobei die Probe ein Arzneimittel, Arzneimittelbestandteil, Nahrungsmittel, Nahrungsmittelbestandteil oder Roh- oder Ausgangsstoff für Nahrungsmittel oder Arzneimitteln ist.

26. Verfahren nach einem der Ansprüche 11 bis 25, wobei in Schritt c) die Inkubation des Probe-Zell-Komplexes zur Induktion bei etwa 37 °C für mindestens etwa 1 Std. und maximal etwa 24 Std. erfolgt.

27. Verwendung der transgenen Zelle nach einem der Ansprüche 1 bis 7 zum spezifischen Nachweis eines Pyrogens in einer klinischen Probe.

28. Verwendung der transgenen Zelle nach einem der Ansprüche 1 bis 7 zum Testen von Erzeugnissen auf Pyrogenfreiheit.

29. Verwendung der transgenen Zelle nach einem der Ansprüche 1 bis 7 zum Screenen von Wirkstoffen mit der Eigenschaft eines Antagonisten für einen humanen TLR.

30. Verwendung der transgenen Zelle nach einem der Ansprüche 1 bis 7 zum Screenen von Oligonucleotiden mit CpG-Motiven.

31. Verwendung nach einem der Ansprüche 27 bis 30, wobei das Kit nach einem der Ansprüche 8 bis 10 eingesetzt wird.

32. Verwendung nach einem der Ansprüche 27 bis 31, wobei das Verfahren nach einem der Ansprüche 11 bis 26 durchgeführt wird.

## Claims

1. Transgenic cell for specific detection of a pyrogen in a sample, containing in the genome:
a) a gene or genes encoding at least one human toll-like receptor (TLR), at least for human TLR-2 and/or human TLR-4 that is expressed by the cell, and
b) at least one reporter gene under expression control by an NF-κB-inducable promoter,
whereby the cell is a murine fibroblast cell of the NIH-3T3-type.

2. Cell according to any one of the preceding claims, whereby the reporter gene encodes a secreted alkaline phosphatase (SEAP).

3. Cell according to any one of the preceding claims, whereby the reporter gene encodes a beta-galactosidase.

4. Cell according to any one of the preceding claims, whereby the reporter gene encodes a luciferase.

5. Cell according to any one of the preceding claims, whereby the reporter gene encodes GFP.

6. Cell according to any one of the preceding claims, whereby the inducable promoter is the promoter for selectin (endothelial cell leukocyte adhesion molecule; ELAM-1).

7. Cell according to any one of the preceding claims expressing, in addition, the type CD14 (MD2) co-receptor.

8. Kit for specific detection of a pyrogen in a sample, containing: culture vessel containing at least one transgenic cell according to any one of the preceding claims.

9. Kit according to claim 8 containing: detection medium, containing substrate for the enzyme encoded by the inducable reporter gene.

10. Kit according to claim 8 or 9 containing: cell culture vessel or cell culture plate having at least two compartments or wells, whereby a first well contains the at least one transgenic cell and a second well contains another cell other than the transgenic cell, expressing at least one other TLR type.

11. Method for specific detection of a pyrogen in a sample, comprising the steps of:
a) providing the sample and at least one transgenic cell according to any one of the claims 1 to 7;
b) contacting the sample and the cell;
c) incubating the sample-cell complex for induction; and
d) detecting the enzyme activity mediated by the induced reporter gene,
whereby the enzyme activity being detected indicates the presence of a corresponding specific pyrogen.

12. Method according to claim 11, whereby step d) comprises the steps of:
d1) providing a detection medium containing substrate for the enzyme encoded by the inducable reporter gene of the cell; and
d2) incubating the induced sample-cell complex in the detection medium for detection of the enzyme activity mediated by the induced reporter gene.

13. Method according to claim 11 or 12, whereby the enzyme activity is an alkaline phosphatase activity.

14. Method according to claim 13, whereby the substrate is 5-bromo-4-chloro-indoylphosphate (BCIP) and the detection is indicated by the colour changing to blue and/or by a blue precipitate.

15. Method according to claim 14, whereby the substrate is p-nitrophenylphosphate (pNPP) and the detection is indicated by the colour of the solution changing to yellow.

16. Method according to claim 15, whereby the colour change of the solution to yellow is quantified by photometry.

17. Method according to claim 11 or 12, whereby the enzyme activity is a beta-galactosidase activity.

18. Method according to claim 17, whereby the substrate is 5-bromo-4-chloro-3-indoyl-beta-D-galactopyranoside (X-Gal) and the detection is indicated by the colour changing to blue and/or by a blue precipitate.

19. Method according to claim 11 or 12, whereby the enzyme activity is a luciferase activity.

20. Method according to claim 19, whereby the substrate is luciferin, possibly containing additional ATP and Mg²⁺, and the detection is indicated by luminescence.

21. Method according to claim 11 or 12, whereby the enzyme activity is GFP.

22. Method according to any one of the claims 11 to 21, whereby the sample is a clinical sample from a human or animal body.

23. Method according to claim 22, whereby the sample is blood.

24. Method according to any one of the claims 11 to 21, whereby the sample is a test part of a medical instrument or medical product.

25. Method according to any one of the claims 11 to 21, whereby the sample is a drug, drug ingredient, food item, ingredient of food items or raw or starting material for food items or drugs.

26. Method according to any one of the claims 11 to 25, whereby the incubation of the sample-cell complex for induction purposes in step c) takes place at approx. 37 °C for at least approx. 1 hour and at most approx. 24 hours.

27. Use of the transgenic cell according to any one of the claims 1 to 7 for specific detection of a pyrogen in a clinical sample.

28. Use of the transgenic cell according to any one of the claims 1 to 7 for the testing of products for the absence of pyrogens.

29. Use of the transgenic cell according to any one of the claims 1 to 7 for the screening of active substances possessing the property of being an antagonist of a human TLR.

30. Use of the transgenic cell according to any one of the claims 1 to 7 for the screening of oligonucleotides with CpG motifs.

31. Use according to any one of the claims 27 to 30, whereby the kit is used according to any one of the claims 8 to 10.

32. Use according to any one of the claims 27 to 31, whereby the method is implemented according to any one of the claims 11 to 26.

## Revendications

1. Cellule transgénique pour l'identification spécifique d'un pyrogène dans un échantillon, contenant dans le génome :
a) un gène ou des gènes codant pour au moins un récepteur de type Toll (TLR) humain, au moins pour TLR-2 humain et/ou TLR-4 humain, qui exprime la cellule, et
b) au moins un gène rapporteur qui est sous le contrôle d'expression d'un promoteur pouvant être induit par NF-κB,
dans laquelle la cellule est une cellule de fibroblaste murin du type NIH-3T3.

2. Cellule selon l'une quelconque des revendications précédentes, dans laquelle le gène rapporteur code pour une phosphatase alcaline secrétée (SEAP).

3. Cellule selon l'une quelconque des revendications précédentes, dans laquelle le gène rapporteur code pour une bêta-galactosidase.

4. Cellule selon l'une quelconque des revendications précédentes, dans laquelle le gène rapporteur code pour une luciférase.

5. Cellule selon l'une quelconque des revendications précédentes, dans laquelle le gène rapporteur code pour une protéine à fluorescence verte.

6. Cellule selon l'une quelconque des revendications précédentes, dans laquelle le promoteur pouvant être induit est le promoteur pour la sélectine (molécule d'adhésion endothélium-leucocyte ; ELAM-1).

7. Cellule selon l'une quelconque des revendications précédentes, qui exprime en outre le co-récepteur de type CD14 (MD2).

8. Kit pour l'identification spécifique d'un pyrogène dans un échantillon, contenant : un récipient de culture avec au moins une cellule transgénique selon l'une quelconque des revendications précédentes.

9. Kit selon la revendication 8, contenant : un milieu de détection contenant du substrat pour l'enzyme codée par le gène rapporteur pouvant être induit.

10. Kit selon la revendication 8 ou 9, contenant : un récipient ou une plaque de culture cellulaire avec au moins deux compartiments ou puits, dans lequel l'au moins une cellule transgénique est contenue dans un premier puits et une autre cellule transgénique différente de la cellule transgénique, exprimant au moins un autre type de TLR, est contenue dans un second puits.

11. Procédé d'identification spécifique d'un pyrogène dans un échantillon, comprenant les étapes de :
a) fourniture de l'échantillon et d'au moins une cellule transgénique selon l'une quelconque des revendications 1 à 7 ;
b) mise en contact de l'échantillon avec la cellule ;
c) incubation du complexe échantillon-cellule pour l'induction ; et
d) détection de l'activité enzymatique développée par le gène rapporteur induit,
dans lequel la détection de l'activité enzymatique annonce la présence d'un pyrogène spécifique à celle-ci.

12. Procédé selon la revendication 11, dans lequel l'étape d) comprend les étapes de :
d1) fourniture d'un milieu de détection contenant du substrat pour l'enzyme codée par le gène rapporteur de la cellule pouvant être induit ; et
d2) incubation du complexe échantillon-cellule induit dans le milieu de détection pour la détection de l'activité enzymatique développée par le gène rapporteur induit.

13. Procédé selon la revendication 11 ou 12, dans lequel l'activité enzymatique est une activité de phosphatase alcaline.

14. Procédé selon la revendication 13, dans lequel le substrat est du 5-bromo-4-chloro-indoylphosphate (BCIP) et la détection est annoncée par une enveloppe colorée bleue et/ou un précipité bleu.

15. Procédé selon la revendication 14, dans lequel le substrat est du p-nitrophénylphosphate (pNPP) et la détection est annoncée par une enveloppe colorée jaune de la solution.

16. Procédé selon la revendication 15, dans lequel l'enveloppe colorée jaune de la solution est quantifiée de manière photométrique.

17. Procédé selon la revendication 11 ou 12, dans lequel l'activité enzymatique est une activité de bêta-galactosidase.

18. Procédé selon la revendication 17, dans lequel le substrat est du 5-bromo-4-chloro-3-indoyl-bêta-D-galactopyranoside (X-Gal) et la détection est annoncée par une enveloppe colorée bleue et/ou un précipité bleu.

19. Procédé selon la revendication 11 ou 12, dans lequel l'activité enzymatique est une activité de luciférase.

20. Procédé selon la revendication 19, dans lequel le substrat est la luciférine, éventuellement en plus avec de l'ATP et Mg²⁺, et la détection est annoncée par luminescence.

21. Procédé selon la revendication 11 ou 12, dans lequel l'activité enzymatique est une protéine à fluorescence verte.

22. Procédé selon l'une quelconque des revendications 11 à 21, dans lequel l'échantillon est un échantillon clinique provenant d'un corps humain ou animal.

23. Procédé selon la revendication 22, dans lequel l'échantillon est du sang.

24. Procédé selon l'une quelconque des revendications 11 à 21, dans lequel l'échantillon est un spécimen d'un instrument médical ou d'un produit médical.

25. Procédé selon l'une quelconque des revendications 11 à 21, dans lequel l'échantillon est un médicament, un constituant de médicament, un aliment, un constituant d'aliment ou une matière première ou de départ pour aliment ou médicaments.

26. Procédé selon l'une quelconque des revendications 11 à 25, dans lequel, à l'étape c), l'incubation du complexe échantillon-celllule pour l'induction s'effectue à environ 37°C pendant au moins environ 1 heure et au maximum environ 24 heures.

27. Utilisation de la cellule transgénique selon l'une quelconque des revendications 1 à 7 pour l'identification spécifique d'un pyrogène dans un échantillon clinique.

28. Utilisation de la cellule transgénique selon l'une quelconque des revendications 1 à 7 pour le test de produits à la recherche d'apyrogénicité.

29. Utilisation de la cellule transgénique selon l'une quelconque des revendications 1 à 7 pour le criblage de substances actives avec la propriété d'un antagoniste pour un TLR humain.

30. Utilisation de la cellule transgénique selon l'une quelconque des revendications 1 à 7 pour le criblage d'oligonucléotides avec des motifs CpG.

31. Utilisation selon l'une quelconque des revendications 27 à 30, dans laquelle le kit selon l'une quelconque des revendications 8 à 10 est utilisé.

32. Utilisation selon l'une quelconque des revendications 27 à 31, dans laquelle le procédé selon l'une quelconque des revendications 11 à 26 est exécuté.
